# EUROPEAN PATENT APPLICATION

(11) **EP 1 621 187 A1**
(43) Date of publication of application: **01.02.2006**
(21) Application number: 04291900.1
(22) Date of filing: 26.07.2004
(51) Int. Cl.: A61K 9/26

(54) **Pharmaceutical multiparticulate tablet formulations and process for their preparation**

(71) Applicant: AstraZeneca AB, 151 85 Södertälje (SE)
(72) Inventor: Gil, Alexander, 06250 Mougins (FR); Combessis, Danielle, 34000 Montpellier (FR); Corvaisier, David, Le Panaroma 06410 Biot (FR); Guerin, Emmanuel, 92700 Colombes (FR)
(74) Representative: Koch, Gustave

(57) **Abstract**

The present invention relates to a multiparticulate tablet with improved gastro-protection comprising at least a pharmaceutically active substance in the form of enteric coated particles, and a mixture of tableting excipients, wherein the said mixture of excipients comprising xylitol and/or maltitol, each in a directly compressible form, a disintegrating agent, a lubricant and at least one other diluent and the ratio of a) the xylitol and/or the maltitol to b) the other diluent(s) is less than 5/95 (weight/weight) and the result of the "test of integrity of the film" is greater than 95%, preferably greater than 97% and more preferably still greater than 99% and the result of the "release test" is greater than 90%, preferably greater than 95%. According to one embodiment of the invention, the active substance is omeprazole or esomeprazole. According to another embodiment the tablet is a disintegratable tablet, which disintegrate in the mouth with or without chewing. The invention also comprises a process for preparing the claim tablet and its use in medicine.

## Description

### Field of the invention.

The present invention relates to a multiparticulate tablet comprising a multiple of small enteric coated pellets or particles of a pharmaceutically active substance. According to one aspect of the invention, the active substance is an acid labile proton pump inhibitor compound. According to another aspect the multiparticulate tablet comprising a pharmaceutically active substance is an orally disintegratable tablet, which disintegrate in the mouth. The present invention also relates to processes for the preparation of the said tablets and their use in medicine.

### Background of the invention.

Pharmaceutically active substances have different physicochemical and biopharmaceutical characteristics, which in some cases require that the release of the substance is target so as to obtain optimal absorption of the active substance in the body, for instance to avoid possible decomposition of the active substance in the acidic stomach. This is specifically true for substances that are sensitive to gastric acidity or the substance need to be released as close as possible to its site of absorption and/or action.

In the present patent application, the terms "enteric polymer", "enteric coating", "gastro-resistant polymer" and "gastro-resistant coating" will be used interchangeable.

The use of an enteric or gastro-resistant polymer allows the Formulator to formulate the active substance so that it remains intact during its passage and/or its time of residence in the acidic gastric juice. The passage of the stomach, which may take approximately up to two hours, includes presence in a medium with a pH of between approximately 1 and 3. Once in the small intestine, which comprises of the duodenum, the jejunum and the ileum, the enteric coating will rapidly dissolve. The small intestine comprises a medium with a pH of greater then 4.5 and which gradually increases up to a pH of approximately 7.2 in its distal part.

Thus, active substances, which are acid labile, will be decomposed by the acidic gastric juice and they require gastro-protection for oral administration. There are some active substances that might be acid stable but irritating the gastric mucosa and therefore benefit from a protective layer. Thus, there might be a need for gastro-protection, i.e. an enteric coating, for different types of pharmaceutically active substance.

For oral administration, it is particularly advantageous to formulate such active substances to multiple unit dosage forms. The active substance is formulated to small particles, such as pellets, micro tablets, spheroids or granules, preferably small spherical particles, see for instance EP -A-247 983, which are covered with a subcoating layer and an outer enteric coating.

The small particles can be composed according to different principles, such as a seed (for instance a sugar sphere) layered with the active substance and/or pharmaceutically acceptable constituents or the active substance is mixed with pharmaceutical acceptable constituents and the particles are produced by extrusion/ spheronisation, balling or compression utilizing suitable process equipment.

The small particles, i.e. a core material comprising the active substance, will be coated with a subcoating layer and an external layer composed of at least one enteric polymer.

The coated particles are subsequently placed in capsules, such as hard gelatine capsules. Alternatively, cellulose capsules for instance capsules of hydroxyl propyl methylcellulose can be used.

In alternatively multiple unit dosage forms, the coated particles are compressed to a tablet after being mixed with pharmaceutically acceptable tablet excipients. When preparing multiple unit tablets, the application of compression forces to the tablet mixture comprising coated particles present a problem with respect to the strength of the coating and specifically the requirement to maintain the gastro-resistance and the integrity of the tablet and of the enteric coated units after tableting. WO 96/01624 discloses a multiple unit tablet comprising gastro- resistant micro-granules of a proton pump inhibitor compound wherein the enteric coating maintain the gastro- resistance and withstand the compression forces during tableting.

It has been shown that the film-forming agents generally used to coat particles cannot under normal conditions absorb the mechanical stresses applied during tableting (International Journal of Pharmaceutics, No. 143, 13-23, 1996). Films composed of only enteric polymers or copolymers have very mediocre mechanical properties, such that they do not withstand tableting. The application of these compression forces can result in the appearance of cracks in the enteric coating film or by the splitting thereof, resulting in the partial or complete loss of the properties of the film coating.

The prior art provides solutions such as for example modifying the composition of the enteric coating films, so as to substantially improve its mechanical characteristics with regard to tableting properties, i.e. to withstand the application of compression forces. Improvements in gastro-resistance and less film damage can also be achieved by using excipients that deform plastically during tableting.

The document "Drugs made in Germany", 37, No. 2 (1994), p. 53, teaches that it is possible to combine Eudragit® L30D and Eudragit® NE30D to produce multiparticulate tablets comprising the said enteric coated particles. However, this approach does not work for all active principles, such as coating of acidic sensible compounds.

WO 02/19991 (Röhm) relates to a multiparticulate tablet and gastro-resistant micro-granules, wherein the said micro-granules comprise an enteric coating of a methacrylic acid copolymer and propylene glycol. The proportion of the said granules in the tablets is between 35% and 90%, preferably 40% to 70%, by weight, with respect to the total weight of the tablet, the remainder being a binder.

Alternative solutions consist of diluting/mixing the enteric coated particles with auxiliary substances, which substances can absorb the physical stresses during tableting. This solution often requires a lengthy formulation operation and is not suited to all types of tablets, such as water-dispersible tablets, or tablets, which disintegrate in the mouth, with or without chewing.

Orally disintegratable tablets, which disintegrate in the mouth, are disclosed for instance in EP 548356, EP 1003484, EP 1126821, EP 1156786, WO 03/007917, WO 98/53798 and WO 00/78292.

EP 1003484 describes a composition of an active ingredient with a taste-masking coating and tablet excipients comprising at least one disintegrating agent and at least one soluble diluent agent with binding properties. WO 98/53798 describes a solid preparation comprising a pharmaceutically active ingredient, one or more water-soluble sugar and low-substituted hydroxyprolpylcellulose having hydroxyl group contents of 7.0 to 9.9 % by weight. WO 00/78292 describes quickly disintegrating solid preparations containing an active ingredient, D-mannitol having an average paricle size of 30µm to 300µm, a disintegrating agent and celluloses.

### Outline of the invention.

It is now surprisingly been found that it is possible, by adding a small amount of a specific diluent to the mixture of tableting excipients, to prepare a multiparticulate tablet comprising a pharmaceutically active substance such as for instance a proton pump inhibitor, exemplified with omeprazole and esomeprazole, in the form of enteric coated particles without the application of tableting forces, which may detrimentally affect the integrity of the coated particles. The multiparticulate tablet of the present invention exhibits an improved gastro-resistance.

Surprisingly and unexpectedly, it has been found that the presence of xylitol and/or of maltitol, in their directly compressible form and in a very small amount, makes it possible to improve the tableting ability of the mixture of tablet's excipients to be tableted and to retain the gastro-protective properties provided by the enteric polymer.

It has been shown that once disintegrated, the coated particles from the multiparticulate tablet of the invention are retained independently and intact. The release profiles obtained from the tablet comprising the enteric coated particles of the pharmaceutically active substance such as a proton pump inhibitor is virtually the same as the release profile from the coated particles as such and before the compression to a tablet.

The presence of xylitol and/or of maltitol, in their directly compressible form and in a very small amount, surprisingly makes it possible to retain organoleptic characteristics and a rate of disintegration which are equivalent to those of tablets which do not comprise these directly compressible sugars. These characteristics are specifically important and essential for water-dispersible tablets and orally disintegratable tablets, which disintegrate in the mouth, with or without chewing of the tablets.

The invention thus relates to a multiparticulate tablet with an improved gastro-protection comprising at least a pharmaceutically active substance in the form of enteric coated particles, and a mixture of tableting excipients, the said mixture of excipients comprising xylitol and/or maltitol, each in a directly compressible form, a disintegrating agent, a lubricant and at least one other diluent. The ratio of a) the xylitol and/or the maltitol to b) the other diluent(s) is less than 5/95 (weight/weight). The result of the "test of integrity of the film" is greater than 95%, preferably greater than 97% and more preferably still greater than 99% and the result of the "release test" is greater than 90%, preferably greater than 95%.

According to one aspect of the invention, the multiparticulate tablet is an orodispersible tablet tablets which are able to disintegrate or dissolve in the buccal cavity, without mastication, upon contact with saliva, in less than 60 seconds and preferably less than 40 seconds, forming a particle suspension that is easy to swallow, said tablet comprising at least a pharmaceutically active substance in the form of enteric coated particles, and a mixture of tableting excipients at least comprising xylitol and/or maltitol, each in a directly compressible form, a disintegrating agent, a lubricant and at least one other diluent, the ratio of a) the xylitol and/or the maltitol to b) the other diluent(s) is less than 5/95 (weight/weight), and optionally a swelling agent, an antistatic agent, a permeabilising agent, sweeteners, flavoring agents and colors.

The disintegration time corresponds to the time between the moment when the tablet is placed on the tongue and the moment that the suspension resulting from the disintegration or dissolution of the tablet is swallowed.

According to another aspect of the invention the pharmaceutically active substance is a proton pump inhibitor. Thus, the invention relates to a multiparticulate tablet with an improved gastro-protection comprising at least one proton pump inhibitor, such as omeprazole or esomeprazole, in the form of enteric coated particles, and a mixture of tableting excipients, the said mixture of excipients comprising xylitol and/or maltitol, each in a directly compressible form, a disintegrating agent, a lubricant and at least one other diluent. The ratio of a) the xylitol and/or the maltitol to b) the other diluent(s) is less than 5/95 (weight/weight). The result of the "test of integrity of the film" is greater than 95%, preferably greater than 97% and more preferably still greater than 99% and the result of the "release test" is greater than 90%, preferably greater than 95%.

The "test of integrity of the film" and the "release test" make it possible to characterize the gastro-resistance of multiparticulate tablets.

These tests are carried out in the following way:

### "Test of integrity of the film":

The enteric pharmaceutical dosage form (according to the present invention, a multiparticulate tablet) is placed for 120 minutes in a dissolution medium with a pH of 1.2. After this period of time the coated active particles are collected.

The amount of active substance remaining within the enteric coating is then assayed, which makes it possible to directly assess the integrity of the insoluble enteric coating film at pH 1.2. Any amount of active substance released into the acidic medium, in the event of a detrimental change of the enteric coating, will be decomposed itself by the medium.

The result is expressed as percentage by weight with respect to the total starting amount of the active substance.

In the case of the multiparticulate tablet of the invention, the latter disintegrates in the medium with a pH of 1.2, releasing the coated active particles, which are then directly in contact with the medium with a pH of 1.2; the amount of the active substance remaining in the enteric coated particles on conclusion of this test being greater than 95%, preferably greater than 97% and more preferably still greater than 99%.

### "Release test":

It consists of the determination of the dissolution profile of the enteric coated particles by a dissolution profile derived from that described, for example, in the United States Pharmacopoeia (US Pharmacopeia, XXth Ed.).

In a first step, the enteric pharmaceutical dosage form is placed for 120 minutes in a dissolution medium with a pH of 1.2. Then, in a second step, this same enteric coated dosage form is placed for 30 minutes in a medium with a pH, which is increased up to a value of 6.8 by addition of an alkaline buffer solution to the medium from the first step.

The amount of the active substance released into the medium with at pH 6.8 after 30 minutes is subsequently measured, which makes it possible to directly assess the integrity of the enteric coated particle itself and to confirm that the active substance is indeed immediately released in the second medium.

The result is expressed as percentage by weight with respect to the total starting amount of the active substance.

In the present invention, the multiparticulate tablet disintegrates in the medium with a pH of 1.2, releasing the enteric coated particles, which are then directly in contact with the medium with a pH of 1.2 (in which the enteric polymer is insoluble). Then the enteric coated particles are in contact with the medium with a pH of 6.8, at which pH the enteric polymer is soluble and at which pH the active substance, thus will be released. With the tablet of the invention, an amount of greater than 90%, preferably of greater than 95%, is thus released after 30 minutes in a dissolution medium with a pH of 6.8.

The invention thus relates to a multiparticulate tablet with improved gastro-protection, comprising at least one pharmaceutically active substance in the form of enteric coated particles, and a mixture of tableting excipients. The said mixture of excipients comprising xylitol or maltitol, each in a directly compressible form, a disintegrating agent, a lubricant and at least one other diluent. The ratio of a) the xylitol and/or the maltitol to b) the other diluent(s) is less than 5/95 (weight/weight), preferably less than or equal to 3/97 (weight/weight), more preferably still approximately 1/99 (weight/weight).

According to one aspect the pharmaceutically active substance is a proton pump inhibitor compound. Thus, according to this aspect the invention relates to a multiparticulate tablet with improved gastro-protection, comprising at least one proton pump inhibitor, such as omeprazole or esomeprazole, in the form of enteric coated particles, and a mixture of tableting excipients. The said mixture of excipients comprising xylitol or maltitol, each in a directly compressible form, a disintegrating agent, a lubricant and at least one other diluent. The ratio of a) the xylitol and/or the maltitol to b) the other diluent(s) is less than 5/95 (weight/weight), preferably less than or equal to 3/97 (weight/weight), more preferably still approximately 1/99 (weight/weight).

In the present invention, powder "in a directly compressible form" is understood to mean a powder for which the mean diameter of the particles is between 100 µm and 500 µm.

Pharmaceutically active substances suitable according to the present invention are:
Types of pharmaceutically active substances might benefit from a gastro-protection and may be chosen from any family of drugs, for example from gastro-intestinal sedatives, antacids, analgesics, anti-inflammatories, coronary vasodilators, peripheral and cerebral vasodilators, anti-infective, antibiotics, antiviral agents, antiparasitic agents, anticancer agents, anxiolytics, neuroleptics, central nervous system stimulants, antidepressants, antihistamines, antidiarrheal agents, laxatives, dietary supplements, immunodepressants, hypocholesterolaemiants, hormones, enzymes, antispasmodics, anti-anginal agents, medicinal products that affect the heart rate, medicinal products used in the treatment of arterial hypertension, antimigraine agents, medicinal products that affect blood clotting, antiepileptics, muscle relaxants, medicinal products used in the treatment of diabetes, medicinal products used in the treatment of thyroid dysfunctions, diuretics, anorexigenic agents, anti-asthmatics, expectorants, antitussive agents, mucoregulators, decongestants, hypnotics, antinausea agents, hematopoietic agents, uricosuric agents, plant extracts, contrast agents or any other family of compounds.

The active substance is provided as such or in the form of a pharmaceutically acceptable salt thereof, and if appropriate in its racemic form or in the form of one of its pure enantiomer or any polymorphic form. With the expression "a pharmaceutically acceptable salt thereof" is meant any basic or acidic salts, such as salts with inorganic or organic acid, such as carboxylic acid; or salts with amines; or salts with alkaline compounds, or similar derivatives.

According to a specific aspect of the present invention pharmaceutically active substances that need gastro-protection for oral administration are suitable, such as acid labile compounds, which inhibit the proton pump. Such substances are for instance omeprazole, lansoprazole, pantoprazole, pariprazole, leminoprazole and rabeprazole. These substances might be provided in their neutral form or in the form of alkali metal salts, in their racemic form or in the form of their pure enantiomers, or in any polymorphic form.

The proton pump inhibitor compound, omeprazole, and its (S)-enantiomer, esomeprazole, are well recognised and sold under the trade names Losec® and Nexium®, respectively.

In the present patent application, the terms "proton pump inhibitor", "PPI" and "compound which inhibits the proton pump" will be used interchangeable to denote any compound of this family. These compounds are present in the neutral form, in the form of an alkali metal salt, in the form of a racemate or of an enantiomer, or in any polymorphic form.

Other types of pharmaceutically active substances suitable according to the present invention are compounds that may irritate the gastric mucosa, such as for instance non-steroidal anti-inflammatory drugs (NSAID), such as diclofenac; antibiotics, such as doxyxycline or erythromycin and derivatives thereof; and also substances that need an administration with delayed release.

The active substance can be provided in the form of a core material that can be prepared from powder or of micro-crystals of the active substance, which may be employed in the form of granules prepared by dry or wet granulation or in the form of pellets or of spheroids prepared by attaching to inert supports and/or extrusion-spheronization.
I) In a first embodiment, the core material comprising the active substance is prepared by granulation according to the following stages:
   - dry mixing the active substance, in the form of a powder or of micro-crystals, optionally with a diluent and an antistatic agent,
   - granulating the mixture obtained by spraying with a solution of the binding agent,
   - drying.

   For the granulation conventional equipment such as a high-energy granulator, a planetary mixer or a fluidized air bed is used.
II) In a second embodiment, the core material comprising the active substance is prepared by attaching the active substance to an inert support such as for instance a sugar sphere according to the following stages:
   - spraying over inert supports a solution or a suspension comprising the active substance, a dissolved binder and, optionally, a lubricant and an antistatic agent,
   - drying.

The composition to be used for the spraying can be provided, according to the circumstances, in the form of a suspension, in the form of a solution, or in the form of an emulsion in an aqueous or organic media or in the molten state.

In a first alternative form of the attaching process, the active substance is incorporated in the attaching composition, which is applied to the inert supports.

According to another alternative form of the attaching process, the active substance is applied by dusting to the inert supports, which are wetted beforehand with the attaching composition.

All process steps of the present invention can be carried out in conventional equipment such as in a coating pan or a perforated pan or in a fluidized air bed.

II) According to a third embodiment, the particles comprising the active substance are prepared by extrusion-spheronization.

The mixture comprising the active substance and the pharmaceutically acceptable excipients is moistened or heated in order to provide satisfactory extrusion, and the extrudates thus obtained are graded and spheronized.

In the present description, the term "core material" or "active particle" is used to denote without distinction one or other forms under which the active substance can be prepared as pellets, granules or micro tablets, which then are coated with an enteric coating polymer.

The inert support can be composed of any chemically and pharmaceutically inert excipient existing in the crystalline or amorphous particulate form, for example sugar derivatives, such as lactose, sucrose, hydrolysed starch (maltodextrin) or celluloses. Mixtures, such as sucrose and starch or mixtures based on cellulose, are also used for the preparation of inert spherical supports. The unit particulate dimension of the inert support can be between 50 µm and 1 000 µm, preferably between 200 µm and 710 µm.

The core material or active particle can additionally comprise one or more excipients chosen from the group consisting of binders, diluents, antistatic agents, and agents for modifying the surrounding micro-pH as well as any mixtures thereof.

The binder is present in proportions, which can range up to 15% by weight, and according to another aspect up to 10% by weight, with respect to the weight of the uncoated particles. The binder can be chosen from the group consisting in particular of cellulose polymers, acrylic polymers, povidones, copovidones, poly(vinyl alcohol)s, alginic acid, sodium alginate, starch, pregelatinized starch, sugars and their derivatives, guar gum, poly(ethylene glycol)s and any mixtures thereof. One role of the binder is to fasten the active substance to the inert supports without loss of material, or to "stick" together the active substance powder or micro-crystals, and the other excipients, in order to give homogeneous core material with an even distribution of the active substance.

The diluent is present in proportions, which can range up to 95% by weight, and according to another aspect up to 50% by weight, with respect to the weight of the core material and can be chosen from the group consisting in particular of cellulose derivatives and preferably microcrystalline cellulose, polyols and preferably mannitol, single starches, sugar derivatives, such as lactose, and any mixtures thereof. One role of the diluent is to increase the total mass of core material, which is to be coated and to produce a population of particles of homogeneous size.

The antistatic agent is present in proportions which can range up to 10% by weight, and according to another aspect up to 3% by weight, with respect to the weight of the uncoated particles and can be chosen from the group consisting in particular of colloidal silica, in particular that sold under the trade name Aerosil®, and according to another aspect precipitated silica, in particular that sold under the name Syloid® FP244, micronized talc, non-micronized talc and any mixtures thereof.

The antistatic agent improves the fluidization of the material when using a fluidized air bed, and in particular in the case of a powder granulation.

The agent for modifying the surrounding micro-pH can be an acidic or basic compound. When the pharmaceutically active substance is an acid labile substance, for example proton pump inhibitor, the agent modifying the micro-pH is always a basic compound.

The acidic agent can be composed of any inorganic or organic acid, in the form of the free acid, of an acid anhydride or of an acid salt.

This acid is chosen from the group consisting in particular of tartaric acid, citric acid, maleic acid, fumaric acid, malic acid, adipic acid, succinic acid, lactic acid, glycolic acid, α-hydroxy acids, ascorbic acid and amino acids, and the salts and derivatives of these acids.

The basic compound is chosen from the group consisting of potassium carbonate, lithium carbonate, sodium carbonate, calcium carbonate, ammonium carbonate, L-lysine carbonate, arginine carbonate, sodium glycine carbonate, sodium carbonates of amino acids, anhydrous sodium perborate, effervescent perborate, sodium perborate monohydrate, sodium percarbonate, sodium dichloroisocyanurate, sodium hypochlorite, calcium hypochlorite and any mixtures thereof. In the context of the present invention, the carbonate is for instance a carbonate, a sesquicarbonate or a hydrogencarbonate.

The amount of agent for modifying the surrounding micro-pH is between 0 and 20%, preferably between 5% and 15% and more preferably between 5% and 10% by weight, with respect to the weight of the uncoated core material or active particles.

One or several optional polymeric layers can be applied between the core material itself and the enteric coating polymer, in order to isolate the active core in the case of possible physicochemical incompatibilities between the components of each of the parts or in order to strengthen the gastro-protection or to improve the stability of the formulation.

Each optional polymeric layer is composed of at least one film-forming polymeric agent chosen from the same polymers as those used as binder. The film-forming polymeric agent is being used either as binding agent or as a separating layer. The relative amount of polymer is, in the first case, less than or equal to 40% by weight, calculated with respect to the dry mass of the optional polymeric layer. In the second case, the relative amount of polymer is greater than 40% by weight, calculated with respect to the dry mass of the said layer.

The optional polymeric layer can additionally comprise one or more protective agents, for example a weakly hygroscopic agent, such as mannitol, an opacifying agent, such as titanium dioxide, or also acidic or basic substances, preferably chosen from those used to locally create a micro-pH, or also hydrophobic substances, such as, for example, mono-and diacetylated glycerides (Myvacet®), silicone oils (Dimethicone®) or triglycerides (Gelucire®).

The optional polymeric coating layer can additionally comprise a plasticizer, a lubricant or adjuvants of the type of those used in the enteric coating layer. In this case, the polymer is chosen from the same polymers as those used as binder.

The amount of polymer applied is between 1% and 10% as increase in weight with respect to the mass of active cores employed, preferably between 2% and 5%.

The core material or active particle comprising the active substance is subsequently coated with a film which provides the gastro-protection of the active substance and which film comprises a film-forming enteric polymer and at least one plasticizer.

The enteric polymer is chosen from the group consisting of cellulose acetate phthalate, hydroxypropylmethylcellulose phthalate, hydroxypropylmethylcellulose succinate phthalate, polyvinyl acetate phthalate, cellulose acetate trimellitate, carboxymethylcellulose, shellac and any other enteric polymer, used alone, as a mixture or combined separately. According to one feature of the invention the enteric polymer is the methacrylic acid copolymers sold under the trade name Eudragit® L100 and Eudragit® L30D.

The enteric coating composition is applied by spraying in order to form a continuous film covering the entire surface of each active particle. The enteric coating layer is applied in an amount sufficient to provide the gastro-protection of the active substance depending on the situation and surface condition of the core material. The enteric coating is present in proportions, which can range up to approximately 50%, and according to another feature up to approximately 20%, calculated as increase in weight with respect to the mass of active particles to be coated.

The solvent chosen for spraying the enteric polymer can be water, an organic solvent, such as ethanol, isopropanol or acetone, or any mixture of these solvents.

The enteric coating polymer is prepared in the form of a solution, suspension or colloidal dispersion in any of the solvent or mixture of solvents. According to a preferred embodiment the enteric coating polymer is prepared in the form of a colloidal dispersion in water.

Optionally, the enteric coating polymer can be mixed with a second polymer or copolymer, which can itself be soluble or insoluble, in particular a neutral copolymer of esters of acrylic acid and of methacrylic acid sold under the trade name Eudragit® NE30D.

The addition of a second polymer to the enteric coating composition makes it possible to improve the mechanical properties of the enteric film resulting from this mixture. In this case, the second polymer is added in an amount of at most 100%, calculated as dry weight of polymer with respect to the dry weight of the enteric polymer, and according to a preferred embodiment in a ratio of between 10% and 30%.

The enteric coating composition can also comprise a plasticizer. The plasticizer is chosen from the group consisting of triethyl citrate, acetyl tributyl citrate, triacetin, tributyl citrate, diethyl phthalate, polyethylene glycols, polysorbates, mono- and diacetylated glycerides, and any mixtures thereof. One role of the plasticizer is to lower the glass transition temperature of the film.

The plasticizer is usually used in a total proportion of at most 40%, preferably between 10% and 30%, expressed by weight with respect to the dry weight of polymer.

The enteric coating composition may also optionally comprise a surface-active agent, an antistatic agent or a lubricant. The surface-active agent is chosen from anionic, cationic, nonionic or amphoteric surfactants.

An antistatic agent is used, for example, to prevent problems related to static electricity. It is chosen from the group consisting of micronized talc, non-micronized talc, colloidal silica (Aerosil® 200), treated silica (Aerosil® R972), precipitated silica (Syloid® FP244) and any mixtures thereof. The antistatic agent is usually used in a proportion of at most approximately 10% by weight, preferably between 0 and 3%, preferably of less than approximately 1% by weight.

The lubricant is chosen from the group consisting of magnesium stearate, stearic acid, sodium stearyl fumarate, micronized polyethylene glycols, sodium benzoate and any mixtures thereof.

The particle size of the coated particles must allow them to be used in the manufacture of multiparticulate tablets. The size is determined by conventional methods, for example using a set of sieves of calibrated mesh size or by laser diffraction. The particle size diameter is between 0.1 and 2 mm, preferably between 0.3. and 1 mm, and more preferably between 0.5 and 1 mm.

In a preferred embodiment of the process for the preparation of the coated particles, all steps for the preparation of the active particles, and coating steps are carried out in a fluidized air bed. The fluidized air bed is equipped with a spray nozzle, in which the position and the direction of spraying can be chosen. The method of spraying is described as top spray, bottom spray of tangential spray, according to the usual terminology of a person skilled in the art.

The choice of the method of spraying makes it possible to control the kinetics of growth of the particles and to prevent phenomena of sticking, related to the nature of the active substance, to the binding or coating composition sprayed and to the various processing parameters (temperature, air pressure, for example, flow rate of solution).

In addition to the particles coated with an enteric polymer, the tablet of the invention comprises a mixture of excipients chosen from the group consisting of at least one diluent, at least one disintegrating agent, at least one lubricant and optionally a swelling agent, an antistatic agent, a binder, an adjuvant and their mixtures, the said mixture of excipients additionally comprising xylitol and/or maltitol, each in a directly compressible form, in a proportion with respect to the amount of the other diluent or diluents of less than 5/95 (weight/weight).

The multiparticulate tablet is preferably an orodispersible tablet comprising a mixture of tableting excipients chosen from the group consisting of at least xylitol and/or maltitol, each in a directly compressible form, at least one disintegrating agent, at least one lubricant and at least one other diluent, the ratio of a) the xylitol and/or the maltitol to b) the other diluent(s) is less than 5/95 (weight/weight), and optionally, tableting excipients chosen from the group comprising swelling agents, antistatic agents, permeabilising agents, sweeteners, flavoring agents and colors.

Advantageously, the ratio of the amount of xylitol and/or the amount of maltitol to the amount of the other diluent or diluents is less than or equal to 3/97, preferably of approximately 1/99.

The xylitol and/or the maltitol are each provided in a directly compressible form, that is to say in the form of a powder for which the mean diameter of the particles is between 100 µm and 500 µm.

The diluent can be chosen from sucrose, lactose, fructose, dextrose, mannitol, sorbitol, lactitol, erythritol, dicalcium phosphate, tricalcium phosphate or microcrystalline cellulose, alone or as a mixture, in a directly compressible form or in the form of a powder for which the mean diameter of the particles is less than 100 µm.

Advantageously, the diluent is mannitol.

The diluent is used in a proportion of between 20% and 90% by weight, preferably between 25% and 60% by weight, calculated with respect to the weight of the tablet.

The disintegrating agent is chosen from the group consisting in particular of crosslinked sodium carboxymethylcellulose, denoted by the term croscarmellose, crosslinked polyvinylpyrrolidones, denoted by the term crospovidone, and their mixtures.

The disintegrating agent is used in a proportion of between 1% and 20% by weight, preferably between 5% and 15% by weight, calculated with respect to the weight of the tablet.

The swelling agent is chosen from the group consisting of microcrystalline cellulose, starches, modified starches, such as sodium starch glycolate or carboxymethylstarch, alginic acid, sodium alginate and their mixtures.

The swelling agent is used in a proportion, which can range up to 20%, preferably of between 1% and 15%, by weight, calculated with respect to the weight of the tablet.

The lubricant is chosen from the group consisting of magnesium stearate, stearic acid, sodium stearyl fumarate, polyethylene glycols, sodium benzoate, pharmaceutically acceptable oil, preferably dimethicone or liquid paraffin, and their mixtures.

The lubricant is always present, either at the heart of the tablet and/or on its surface; it is used in a proportion which can range up to 2%, preferably between 0.02% and 2%, by weight, more preferably between 0.5% and 1% by weight, calculated with respect to the weight of the tablet.

The antistatic agent can be chosen from the group consisting of micronized talc, nonmicronized talc, colloidal silica (Aerosil® 200), treated silica (Aerosil® R972), precipitated silica (Syloid® FP244) and their mixtures.

The antistatic agent is used in a proportion, which can range up to 5% by weight, calculated with respect to the weight of the tablet.

The binder is used in the dry form and can be a starch, a sugar, polyvinylpyrrolidone or carboxymethylcellulose, alone or as a mixture.

The binder is used in a proportion, which can range up to 15% by weight, preferably of less than 10% by weight, calculated with respect to the weight of the tablet.

Adjuvants can also be added to the mixture intended to be tableted and are chosen from the group consisting of pH-adjusting agents, systems which make it possible to produce effervescence, in particular carbon dioxide generators of the type of those used as pH-adjusting agents, surfactants, sweeteners, flavourings, colorants and mixtures.

The preparation of the multiparticulate tablet comprising the enteric particles can comprise the following stages:
- dry mixing the coated active particles with the tableting excipients,
- tableting the mixture in order to obtain a unit form.

In an advantageous method for the preparation of the tablet, the mixing stage itself comprises two stages, the first stage, consisting in mixing the coated active particles with all the tableting excipients except the internal lubricant, and then a second stage, in which the lubricant is added to the first mixture, in all or in part.

In the latter case, the remaining fraction of the lubricant is sprayed over the walls of the die and punches during tableting, the lubricant then being in the form of a powder, for example magnesium stearate, or of a liquid, for example liquid paraffin.

The amounts of lubricant used in the internal and/or external phase are adjusted with care so as to prevent an excess from detrimentally affecting the cohesion of the powder bed during the tableting, leading to capping effector breakage within the tablet.

In another advantageous embodiment, all the lubricant is sprayed over the punches and/or over the internal face of the tableting dies; the second stage of the mixing is then, of course, suppressed.

The tableting of the mixture can be carried out on an alternating or rotary tableting machine.

The stresses during the tabletting stage can vary from 5 kN to 50 kN and preferably between 5 kN and 15 kN.

The hardness of these tablets is preferably between 1 and 10 kp, more preferably between 1 and 5 kp, measured according to the method of the European Pharmacopoeia (2.9.8), 1 kp being equal to 9.8 N.

Preferably, the hardness of the multiparticulate tablet is adjusted so as to obtain friability, measured according to the method of the European Pharmacopoeia, of less than 2%.

The tablet can be round, oval or oblong in shape, can exhibit a flat, concave or convex surface and can optionally exhibit engravings or be bevelled.

The tablet generally has a mass of between 0.1 gram and 2.0 grams and a size in diameter of between 6 mm and 18 mm.

A process for preparing the tablets according to the present invention comprises the following steps:
- preparing of the active particles, i.e. particles comprising the active substance,
- apply an enteric coating on the active particles, optionally after applying a separating layer on the active particles,
- mixing the enteric coated particles with a mixture of tablet excipients comprising xylitol or maltitol, each in a direct compressible form, a disintegrating agent, a lubricant and at least one other diluent, optionally together with at least one additional excipient selected from a swelling agent, an antistatic agent, a binder, an adjuvant and mixtures thereof
- optionally introduce the lubricant in the tablet machine, and
- compress the mixture of enteric coated particle and mixture of tablet excipients to a tablet.

The examples below illustrate the invention.

### Excipients used

Directly compressible mannitol: Parteck M300®, sold by Merck.
Directly compressible xylitol: Xylisorb 300®, sold by Roquette.
Directly compressible maltitol: Maltisorb P200®, sold by Roquette.
Directly compressible sorbitol: Neosorb P60W®, sold by Roquette.
Crospovidone: Kollidon® CL, sold by BASF.
Aspartame: sold by Nutrasweet.
Potassium acesulfame: sold by Sunnett.
Orange and peppermint flavouring: sold by Firmenich.
Magnesium stearate: sold by Peter Graven.
Citric acid: sold by Jungbunzlauer.

### Equipment

The mixer is an inversion mixer with multiple axes of rotation with a capacity of 0.5 to 2 1 supplied by Turbula.

The tableting machine is a SVIAC rotary press equipped with 6 stations of type B for tableting.

### Methods

- Hardness: Schleuniger durometer device, method EP, 4th Ed., 2.9.8,
- Test of integrity of the film: Type II paddle device, USP, 100 revolutions/minute, 500 ml 0.1N HCl medium (pH 1.2),
- Release test: type II paddle device, USP, 100 revolutions/minute, 300 ml 0.1N HCl medium (pH 1.2), then 1 000 ml pH 6.8 buffer (by addition to the medium of 700 ml of Na₂HPO₄·2H₂O),
- HPLC (S)-omeprazole assay: UV detection at 302 nm. **I presume that you are checking (S)-omeprazole?**

### Example 1

The microgranules, coated with an enteric polymer, comprise magnesium salt of the (S)-omeprazole (esomeprazole Mg) as active substance.

These microgranules are manufactured according to the teaching of the prior art (WO 96/01623) to produce a stable formulation. The content of WO 96/01623 is hereby incorporated by reference. The coated micro-granules are prepared according to Example 2 of WO 96/01623, by re-placing omeprazole Mg with (S)-omeprazole Mg.

### 1) Mixtures

The mixture of powders is prepared according to the formulation in Table 1.

**Table 1**

| Formulations (% w/w) | A | B | C |
|---|---|---|---|
| Coated pellets* | 32.2 | 32.2 | 32.2 |
| Mannitol | 53.8 | 53.2 | 51.1 |
| Xylitol | - | 0.6 | 2.7 |
| Crospovidone | 10.0 | 10.0 | 10.0 |
| Aspartame | 0.5 | 0.5 | 0.5 |
| K acesulfame | 1.0 | 1.0 | 1.0 |
| Peppermint flavouring | 1.5 | 1.5 | 1.5 |
| Magnesium stearate | 1.0 | 1.0 | 1.0 |

| | | | |
|---|---|---|---|
| *Coated pellets of (S)-omeprazole Mg | | | |

Three types of mixtures were prepared, each differing in their respective composition of diluent: the first batch (A) does not comprise xylitol, the batch (B) comprises directly compressible xylitol in a ratio by weight of 1/99 with respect to the mannitol 300M, and batch (C) comprises directly compressible xylitol in a ratio by weight of 5/95 with respect to the mannitol.

A first mixture, comprising all the constituents of the formulation except magnesium stearate and in the proportions given in Table 1, is prepared over 5 min at 30 revolutions per minute.

The lubricant is added to the mixture thus obtained by mixing (lubricating stage) for 1 minute at the rate of 30 revolutions/minute.

### 2) Tableting

The mixture obtained in the preceding stage is introduced into the feed hopper of the tableting machine; itself equipped with round, flat and bevelled punches with a diameter of 12 mm.

The tablets prepared have a theoretical mass of 650 mg and comprise a dose of 40 mg calculated as esomeprazole.

These tablets have the following physical and chemical characteristics (Table 2):

**Table 2**

| | A | B | C |
|---|---|---|---|
| Weight (mg) (n=10) | 651.7 | 652.7 | 645.7 |
| Hardness (N) (n=10) | 45 | 45 | 42 |
| Test of integrity of the film (% w/w) | 92.2 | 99.6 | 91.9 |
| Release test (% w/w) | 84.4 | 91.4 | 85.6 |

### 3) Conclusions

The formulation (B), comprising the xylitol in a ratio by weight of 1/99 with respect to the mannitol, exhibits a better suitability for tableting than the formulations (A) and (C); the test of integrity of the film makes it possible to confirm the absence of detrimental change in the enteric coating film, the amount of (S)-omeprazole released and decomposed in a medium with a pH of 1.2 being less than 1% w/w.

The gastro-resistance properties of the multiparticulate tablet are therefore improved.

On the other hand, this effect is not observed for the formulation (C) comprising a xylitol/mannitol ratio of 5/95.

### Example 2

Four tablet formulations were prepared as in Example 1 above using the components given in Table 3 below.

**Table 3**

| Formulations (% w/w) | D | E | F | G |
|---|---|---|---|---|
| Coated (S)-omeprazole Mg | 31.5 | 31.5 | 31.5 | 31.5 |
| Mannitol | 53.5 | 53.5 | 53.5 | 53.5 |
| Xylitol | - | 0.5 | - | - |
| Sorbitol | - | - | 0.5 | - |
| Maltitol | - | - | - | 0.5 |
| Crospovidone | 10.0 | 10.0 | 10.0 | 10.0 |
| Aspartame | 0.5 | 0.5 | 0.5 | 0.5 |
| K acesulfame | 1.0 | 1.0 | 1.0 | 1.0 |
| Flavouring | 1.6 | 1.6 | 1.6 | 1.6 |
| Citric acid | 0.4 | 0.4 | 0.4 | 0.4 |
| Precipitated silica | 0.5 | - | - | - |
| Magnesium stearate | 1.0 | 1.0 | 1.0 | 1.0 |

The physicochemical properties of the tablets thus obtained were measured as in Example 1 and the results are given in Table 4.

**Table 4**

| | D | E | F | G |
|---|---|---|---|---|
| Weight (mg) (n= 10) | 651.4 | 650.5 | 647.5 | 647.6 |
| Hardness (N)(n=10) | 47 | 46 | 48 | 46 |
| Test of integrity of the film (% w/w) | 92.5 | 100.0 | 88.8 | 99.0 |
| Release test (% w/w) | - | 99.9 | 89.8 | 88.6 |

### 3) Conclusions

The formulations (E) and (G), respectively comprising the xylitol and the maltitol in a ratio by weight of 1/99 with respect to the mannitol, exhibit a better suitability for tabletting than the formulations (D) and (F); the test of integrity of the film makes it possible to confirm the absence of detrimental change in the enteric coating film, the amount of (S)-omeprazole released and decomposed in a medium with a pH of 1.2 being less than 1% w/w.

Their gastro-resistance properties are therefore improved.

On the other hand, this effect is not observed for the formulation (F), comprising sorbitol in a sorbitol/mannitol ratio of 1/99.

## Claims

**1.** A multiparticulate tablet comprising at least a pharmaceutically active substance in the form of enteric coated particles, and a mixture of tableting excipients, wherein the said mixture of excipients comprising xylitol and/or maltitol, each in a directly compressible form, a disintegrating agent, a lubricant and at least one other diluent and the ratio of a) the xylitol and/or the maltitol to b) the other diluent(s) is less than 5/95 (weight/weight) and the result of the "test of integrity of the film" is greater than 95%, preferably greater than 97% and more preferably still greater than 99% and the result of the "release test" is greater than 90%, preferably greater than 95%.

**2.** A multiparticulate tablet according to claim 1 wherein the pharmaceutically active substances is chosen from gastrointestinal sedatives, antacids, analgesics, anti-inflammatories, coronary vasodilators, peripheral and cerebral vasodilators, anti-infectives, antibiotics, antiviral agents, antiparasitic agents, anticancer agents, anxiolytics, neuroleptics, central nervous system stimulants, antidepressants, antihistamines, antidiarrheal agents, laxatives, dietary supplements, immunodepressants, hypocholesterolaemiants, hormones, enzymes, antispasmodics, anti-anginal agents, medicinal products that affect the heart rate, medicinal products used in the treatment of arterial hypertension, antimigraine agents, medicinal products that affect blood clotting, anti-epileptics, muscle relaxants, medicinal products used in the treatment of diabetes, medicinal products used in the treatment of thyroid dysfunctions, diuretics, anorexigenic agents, anti-asthmatics, expectorants, antitussive agents, mucoregulators, decongestants, hypnotics, antinausea agents, hematopoietic agents, uricosuric agents, plant extracts, contrast agents.

**3.** A multiparticulate tablet comprising at least one proton pump inhibitor in the form of enteric coated particles, and a mixture of tableting excipients, wherein the said mixture of excipients comprising xylitol and/or maltitol, each in a directly compressible form, a disintegrating agent, a lubricant and at least one other diluent and the ratio of a) the xylitol and/or the maltitol to b) the other diluent(s) is less than 5/95 (weight/weight) and the result of the "test of integrity of the film" is greater than 95%, preferably greater than 97% and more preferably still greater than 99% and the result of the "release test" is greater than 90%, preferably greater than 95%.

**4.** A multiparticulate tablet comprising at least one pharmaceutically active substance in the form of enteric coated particles, and a mixture of tableting excipients, wherein the said mixture of excipients comprising xylitol or maltitol, each in a directly compressible form, a disintegrating agent, a lubricant and at least one other diluent and the ratio of a) the xylitol and/or the maltitol to b) the other diluent(s) is less than 5/95 (weight/weight).

**5.** A multiparticulate tablet according to any of claims 1 and 4 wherein the ratio of a) the xylitol and/or the maltitol to b) the other diluent(s) is less than 3/97 (weight/weight).

**6.** A multiparticulate tablet according to claim 5, wherein the ratio of a) the xylitol and/or the maltitol to b) the other diluent(s) is approximately 1/99 (weight/weight).

**7.** A multiparticulate tablet comprising at least one proton pump inhibitor in the form of enteric coated particles, and a mixture of tableting excipients, wherein the said mixture of excipients comprising xylitol or maltitol, each in a directly compressible form, a disintegrating agent, a lubricant and at least one other diluent and the ratio of a) the xylitol and/or the maltitol to b) the other diluent(s) is less than 5/95 (weight/weight).

**8.** A multiparticulate tablet according to any of claims 3 and 7 wherein the ratio of a) the xylitol and/or the maltitol to b) the other diluent(s) is less than 3/97 (weight/weight).

**9.** A multiparticulate tablet according to claim 8, wherein the ratio of a) the xylitol and/or the maltitol to b) the other diluent(s) is approximately 1/99 (weight/weight).

**10.** A multiparticulate tablet according to any of claims 3 and 7- 9, wherein the proton pump inhibitor is omeprazole or esomeprazole, in neutral form or in the form of an alkaline salt thereof, preferably a magnesium salt of omeprazole or esomeprazole.

**11.** A multiparticulate tablet according to any of claims 1 to 10, wherein the mixture of tablet excipients comprises at least one the of the following additional agents, a swelling agent, an antistatic agent, a binder, an adjuvant and mixtures thereof.

**12.** A multiparticulate tablet according to any of claims 1 to 11, wherein the enteric coating is chosen from the group consisting of cellulose acetate phthalate, hydroxypropylmethylcellulose phthalate, hydroxypropylmethylcellulose succinate phthalate, polyvinyl acetate phthalate, cellulose acetate trimellitate, carboxymethylcellulose, shellac and any other enteric polymer, used alone, as a mixture or combined separately.

**13.** A multiparticulate tablet according to any of claims 1 to 12, wherein the enteric coating is applied in an amount up to approximately 50%, and preferably up to approximately 20%, calculated as increase in weight with respect to the mass of active particles to be coated.

**14.** A multiparticulate tablet according to any of claims 1 to 13, which comprises a mixture of excipients chosen from the group consisting of at least one diluent, at least one disintegrating agent, at least one lubricant and optionally a swelling agent, an antistatic agent, a binder, an adjuvant and their mixtures, the said mixture of excipients additionally comprising xylitol and/or maltitol, each in a directly compressible form, in a proportion with respect to the amount of the other diluent or diluents of less than 5/95 (weight/weight).

**15.** A multiparticulate tablet according to any of claims 1 to 14, which an orodispersible tablet.

**16.** Orodispersible tablet according to claim 15, which comprises a mixture of tableting excipients chosen from the group consisting of at least xylitol and/or maltitol, each in a directly compressible form, at least one disintegrating agent, at least one lubricant and at least one other diluent,

**17.** Orodispersible tablet according to claim 15 or 16, further comprising tableting excipients chosen from the group comprising swelling agents, antistatic agents, permeabilising agents, sweeteners, flavoring agents and colors

**17.** A multiparticulate tablet according to any of claims 1 to 16, wherein the disintegrating agent is present in an amount of 1% to 20% by weight, preferably 5% to 15% by weight, calculated with respect to the total tablet weight.

**18.** A multiparticulate tablet according to any of claims 1 to 17, wherein the diluent is present in an amount of 20% to 90% by weight, preferably 25% to 60% by weight, calculated with respect to the total tablet weight.

**19.** A process for the preparation of a multiparticulate tablet as defined in any of claims 1 to 18, wherein the process comprises the following steps:
- preparing of the active particles, i.e. particles comprising the active substance,
- apply an enteric coating on the active particles, optionally after applying a separating layer on the active particles,
- mixing the enteric coated particles with a mixture of tablet excipients comprising xylitol or maltitol, each in a direct compressible form, a disintegrating agent, a lubricant and at least one other diluent, optionally together with at least one additional excipient selected from a swelling agent, an antistatic agent, a binder, an adjuvant and mixtures thereof
- optionally introduce the lubricant in the tablet machine, and
- compress the mixture of enteric coated particle and mixture of tablet excipients to a tablet.

**20.** A multiparticulate tablet as defined in any of claims 1 to 18 for use in medicine.

**21.** Use of a multiparticulate tablet as defined in any of claims 3 and 7 to 18 in the treatment of gastrointestinal diseases.
